# NOUVEAU FASCICULE DE BREVET EUROPEEN

(11) **EP 0 719 152 B2**
(45) Date de publication et mention de la décision concernant l'opposition: **08.04.2009**
(45) Mention de la délivrance du brevet: 07.01.2004
(21) Numéro de dépôt: 95923374.3
(22) Date de dépôt: 13.06.1995
(51) Int. Cl.: A61K 35/30, A61P 25/00

(54) **NOUVELLES UTILISATIONS D'UN COMPLEXE A BASE DE PHOSPHOLIPIDES CEREBRAUX EN THERAPEUTIQUE ET DANS L'ALIMENTATION**
NEUARTIGE THERAPEUTISCHE UND DIÄTETISCHE VERWENDUNGEN DES AUF PHOSPHOLIPIDEN DER HIRNSABSTANZ BASIERENDEN KOMPLEXES
NOVEL THERAPEUTIC AND DIETETIC USES OF A BRAIN PHOSPHOLIPID-BASED COMPLEX

(30) Priorité: 27.06.1994 FR 9407867
(43) Date de publication de la demande: 03.07.1996
(73) Titulaire: INSTITUT DE RECHERCHE BIOLOGIQUE, F-78870 Bailly (FR)
(72) Inventeur: PONROY, Yves, F-78000 Versailles (FR)
(74) Mandataire: Burtin, Jean-François
(86) Numéro de dépôt international: PCT/FR1995/000771
(87) Numéro de publication internationale: WO 1996/000077

(56) Documents cités:
- EP-A- 0 396 081
- EP-A- 0 661 056
- WO-A-93/21190
- WO-A-94/05319
- BE-A- 693 159
- US-A- 4 385 053
- DATABASE WPI Section Ch, Week 8711 Derwent Publications Ltd., London, GB; Class B04, AN 87-076431 & JP,A,62 029 951 ( SHOWA YAKUHIN KAKO) , 7 Février 1987
- LAROUSSE MEDICALE ILLUSTREE, Dr Galtier-Boissière, Ed. 1922, Librairie Larousse, Paris, p. 193.

## Description

La présente invention se rapporte à de nouvelles utilisations d'un mélange de phospholipides d'origine animale en thérapeutique.

La présente invention a plus particulièrement pour objet l'utilisation d'un mélange de phospholipides extraits de cervelle de mammifères pour la réalisation de préparations à visée thérapeutique.

Elle a spécifiquement pour objet l'utilisation thérapeutique d'une préparation à base de phospholipides cérébraux extraits de cervelles de porc en vue de compenser ou de ralentir les phénomènes de vieillissement et plus précisement la détérioration mentale, l'hypoxie et le vieillissement cérébral.

Le vieillissement physiologique est accompagné au niveau cérébral :
- d'une diminution du taux de phospholipides avec apparition de lysodérivés, et ainsi le rapport cholestérol/phospholipide est augmenté, ce qui entraîne une diminution de la fluidité membranaire (G. CALDERINI dans Phospholipids in the nervous system Vol.2 (1985) 11-18 - Raven Press)
- en outre, les phospholipides membranaires subissent au cours du vieillissement des phénomènes de péroxydations qui altèrent l'activité biologique de ces molécules polyinsaturées (C. DEBY - La biochimie de l'oxygène - La Recherche 22 (1991) 57-64)
- chez l'animal, il a été en effet observé une augmentation du taux de phospholipase cérébrale au cours du vieillissement et une diminution de la capacité de synthèse des phospholipides (A. GAITI - Phospholipids in the nervous system Vol.2 (1985) 155-161 - Raven Press)
- enfin, on sait que chez le sujet âgé, on enregistre une diminution de l'activité de la delta-6-désaturase, qui permet la transformation des acides gras essentiels (18:2 n-6 et 18:3 n-3) en acides gras supérieurs et en particulier en DHA (22:6 n-3) (JM BOURRE (1990) 25 354-356)

Dans la maladie d'Alzheimer, on assiste à des phenomènes du même ordre, mais pas totalement identiques.
- il se forme des pigments lipochromes intracellulaires qui contiennent entre autres, des triglycérides, des céphalines, des sphingomyélines et des acides gras polyinsaturés oxydés (H. DABADIE - Cahiers Nut. Diet. XXII (1987) 51-53). Il pourrait s'agir de molécules de phospholipides cérébraux dégradées.
- l'altération du métabolisme des phospholipides dans la maladie d'Alzheimer, ainsi que les altérations membranaires ont d'ailleurs été mises en évidence par G.ZUBENKO (Brain Research 385 (5) (1986) 115-121).
- on trouve également dans les cerveaux des malades une quantité importante de phosphomono et di-ester, ce qui peut signifier soit une lyse, soit un effort de régénération des phospholipides membranaires (J.W PETTEGREW - J. Neuropath. Exp. Neurology (1987) 46 419-430).
- des études de diffraction aux rayons X montrent une désorganisation de la myéline dûe à une péroxydation des lipides insaturés (L.S CHIA - Biochim Biophys.Acta 775 (1984) 308-312)
- plusieurs études cliniques en double aveugle contre placebo mettent en évidence l'efficacité de la phosphatidylsérine cérébrale à la dose de 300 mg/jour (L. AMADUCCI - Ann. NY Acad. Sci. 640 (1991) 245-249).

Dans l'ischémie cérébrale et les états d'hypoxie expérimentale. on note une augmentation importante du pool d'acides gras libres et, en particuiier. des acides en 20:4 et 22:6. une augmentation des lysodérivés et des diglycérides. De même, plusieurs auteurs font état d'une augmentation du taux de péroxydation des acides gras insaturés. Cette accumulation d'acides gras libres et de composés oxydés altère les fonctions cérébrales (RV DORMAN ( Phospholipids in the nervous system Vol. 1 (1982) 123-135).

Par ailleurs, on sait depuis longtemps, que l'hypoxie cérébrale entraine une baisse importante du métabolisme des phospholipides cérébraux (D.A CHTVERIKOV - Nature 212 (1966) 1236-1238 et Y.DJORRIN - Nature 212 (1966) 1239-1240)

BE- 693 159 se rapporte à la préparation d'un extrait de mammifère en milieu aqueux pour obtenir un extrait de cerveau soluble dans l'eau renfermant en particulier des corps puriques, pyrimidiques et des traces d'acides aminés.

WO 93/21 190 divulgue l'utilisation d'un extrait de cerveau de mammifère comme medicament des neuropathies périphériques.

EP 661 056 décrit l'utilisation de compositions alimentaires de phospholipides cérébraux de porc.

L'ensemble de ces éléments montre que les détériorations mentales provoquées par les états hypoxiques sont associées à une dégradation des phospholipides membranaires.

On a donc étudié l'intérêt d'un apport exogène de phospholipides cérébraux dans le but de régénérer les membranes cellulaires ou de freiner leur dégradation lors de processus physiologiques ou pathologiques et d'apporter au cerveau les acides gras spécifiques et, en particulier, les acides en 20:4 n-6 et 22:6 n-3.

Les phospholipides cérébraux sont caractérisés par le fait qu'ils contiennent une grande proportion d'acides gras à longues chaînes, en particulier de la série n-3. Ils représentent 5% du poids du cerveau et y jouent un rôle structurel et métabolique considérable au niveau membranaire (Lipids malnutrition and the developing brain Elsevier 1972).

La préparation contient un mélange de phospholipides cérébraux en proportion physiologique telle que :

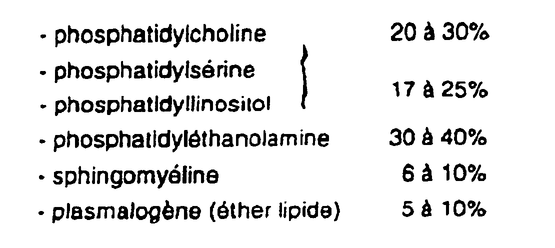

Les phospholipides cérébraux ont ceci de particulier, qu'ils sont constitués de phosphoglycérol lié à deux chaines d'acides gras par une fonction ester ou une fonction éther. Les éthers phospholipidiques, dénommés aussi plasmalogènes, représentent de 5 à 10% de l'ensemble.

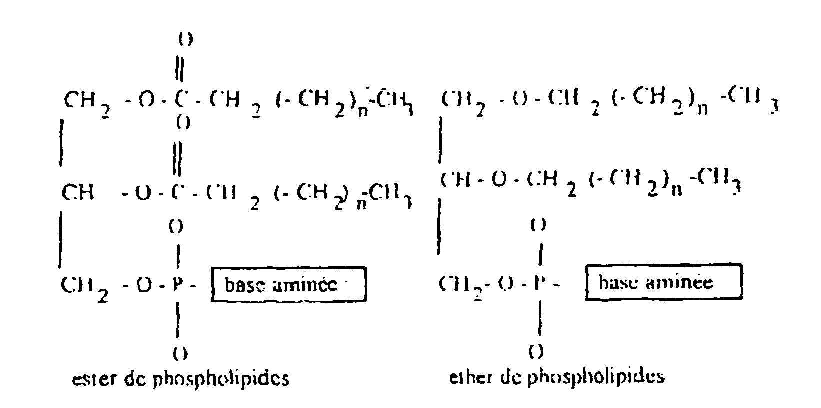

Les phospholipides cérébraux ont, en outre, en particulier, qu'ils sont liés à des acides gras dont la distribution est la suivante, et spécifique par rapport à d'autres phospholipides et, en particulier, par rapport aux phospholipides de soja :
- acide arachidonique 20:4 n-6 7 à 9%
   22:4 n-6 3 à 5%
- DHA 22:6 n-3 7 à 10 %
- EPA 20:5 n-3 < 1%

Les Demandeurs ont, pour cela, réalisé une étude avec des phospholipides marqués, qui a pu mettre en évidence une migration préférentielle des phospholipides cérébraux vers le cerveau après une administration per os.

Les demandeurs ont pu également montrer que des phospholipides cérébraux riches en acides gras à longues chaînes polyinsaturés, administrés per os à des souris, se fixent en partie au niveau cérébral et, en particulier, dans les membranes synaptiques.

Par ailleurs, plusieurs études cliniques réalisées chez l'homme dans divers types de lésions cérébrales, sont venues confirmer l'intérêt de l'utilisation des phospholipides cérébraux dans ces indications.
- dans 20 cas d'insuffisance circulatoire cérébrale, traités avec un produit contenant uniquement des phospholipides cérébraux à raison de 20 à 40 mg/jour pendant une moyenne de 7 mois, il a été observé :
   - une amélioration nette dans 73% des cas aux tests psychométriques WESHLER, REY et BENTON
   - les améliorations les plus nettes, ont porté sur la mémorisation et la composante anxieuse fréquente chez ces malades
   - la tolérance a été excellente
- dans une étude contrôlée contre placebo dans le traitement de la présénescence chez 76 patients d'un âge moyen de 60 ans, l'amélioration sur des critères subjectifs a été statistiquement significative après 3 mois de traitement. en faveur des phospholipides cérébraux (40 mg/jour en deux prises). Les meilleurs résultats sont observés dans les troubles de la mémoire, les troubles de l'attention et de l'efficacité intellectuelle ainsi que l'humeur et l'affectivité La tolérance clinique et biologique a été excellente.

On a essayé, en outre, un produit associant phospholipides cérébraux, anti-oxydants naturels et de l'huile de poisson dans les troubles du vieillissement cérébral, chez 61 sujets. Après 3 mois de tracement. les expérimentateurs ont enregistré une amélioration nette dans 60 à 75% des cas, dans les items suivants sur "l'échelle d'appréciation clinique en gériatrie" (EACG) :
- mémoire des événements récents
- stabilité émotionnelle
- fatigue
- vivacité d'espnt
- anxiété
- initiative
- dépression

La tolérance clinique a été excellente

Enfin, un essai en ouvert chez 23 malades porteurs de séquelles psycho-intellectuelles de traumatismes crâniens, traités pendant 3 mois avec 30 mg/jour de phospholipides cérébraux, a permis d'enregistrer une amélioration globale de l'ordre de 63% au test de BENTON. La mémoire et la fatigabilité sont les plus influencées.

Ceci prouve donc l'intérêt et l'efficacité des préparations à base de phospholipides de cervelles de porc grâce à leur teneur élevée en acides gras essentiels des séries n-3 et n-6. Il en résulte une amélioration des troubles liés au vieillissement cérébral comme la fatigabilité, la perte d'attention ou la difficulté de l'idéation.

Les phospholipides de cervelles de porc sont utilisées à des fins thérapeutiques, seuls ou associés à des excipients, des diluants ou des véhicules permettant leur administration par voie per os ou par voie intraveineuse. La teneur en phospholipides cérébraux peut varier dans des proportions très larges, s'échelonnant de 10 mg à 500 mg par prise unitaire.

Ces phospholipides cérébraux purifiés peuvent être utilisés seuls ou associés. Ils sont destinés essentiellement à la voie digestive, mais peuvent aussi être utilisés par voie générale :
- per os: sous forme de gélules ou capsules à raison de 40 à 400 mg/jour de phospholipides cérébraux
Ils peuvent être utilement associés à des anti-oxydants naturels comme la vitamine C, la vitamine E, le bêta-carotène, des anti-radicalaires comme le sélénium ou la cystéine, des huiles de poissons riches en EPA et DHA
- intraveineuse: sous forme de suspension aqueuse ou de liposomes

Comme anti-oxydants naturels, on pourra citer l'acide ascorbique et ses sels. le palmitate d'ascorbyle et les tocophérols.

Comme agent anti-radicalaire, on pourra citer les dérivés soufrés comme la cystéine, la carboxyméthyl cystéine, le diterbutyl para crésol ou bien encore le métabisulfite de potassium.

Les phospholipides cérébraux selon l'invention sont obtenus par épuisement de cervelles de porc prélevées sur des animaux fraîchement abattus selon les modalités suivantes :
- les cervelles de porc sont prélevées sur des animaux fraîchement abattus en provenance d'élevages exempts de toutes maladies infectieuses et suivis rigoureusement sur le plan sanitaire par les services vétérinaires.
- les cervelles sont immédiatement congelées à -20°C et conservées à cette température.
- les cervelles sont ensuite ramenées à une température comprise entre -5° et 0°, avant passage dans un hâchoir industriel et des broyeurs, de façon à obtenir une pâte liquide dont la teneur en eau est d'environ 80%.
- la pâte de cervelle est transférée au sommet d'une chambre d'atomisation dans laquelle l'eau est immédiatement évaporée dans un courant d'air chaud à 190/195°C.
- la poudre obtenue est introduite dans un réacteur contenant un mélange d'hydrocarbures aliphatiques à base d'hexane et maintenue sous agitation.
- après filtration, la phase liquide est concentrée sous vide et donne naissance à un extrait brut.
- l'extrait brut est ensuite coulé dans l'acétone en présence d'un anti-oxydant alimentaire.
- le précipité obtenu est filtré sous pression d'azote.
- le produit recuerlli est séché sous vide et contient les phospholipides cérébraux purifiés.

Les exemples suivants illustrent l'invention, sans la limiter en aucune façon :

### EXEMPLE I

### Gélules de phospholipides de cervelles de porc

- Phospholipides cérébraux 200 g
- Palmitate d'ascorbyle 12 g
- Vitamine E 60 g
- Sorbitol 40 g
- Gluconate de calcium 25 g
- Stéarate de magnésium 15 g
pour 1 000 gélules

### EXEMPLE II

### Gélules de phospholipides de cervelles de porc

- Phospholipides cérébraux 100 g
- Carbonate de calcium 40 g
- Carbonate de magnésium 30 g
- Phosphate de magnésium 30 g
- Silice colloïdale 25 g
- Vitamine E 25 g
- Talc 15 g
pour 1 000 gélules

### EXEMPLE III

### Gélules de phospholipides de cervelle de porc

- Phospholipides cérébraux 100 g
- Cellulose microcristalline 50 g
- Ethylcellulose 15 g
- Huile de germe de blé 10 g
- Phosphate tricalcique 50 g
- Talc 10 g

### EXEMPLE IV

### Suspension aqueuse de phospholipides cérébraux

- Phospholipides cérébraux 10 g
- Lécithine de soja 5 g
- Vitamine E 1 g
- Eau purifiée qsp 1000 ml

### EXEMPLE V

### Farine alimentaire à base de phospholipides cérébraux

- Phospholipides cérébraux 20 g
- Caséine 5 g
- Méthylcellulose 4,5 g
- Farine de bananes 71,5 g
- Vanilline 0.5 g

## Revendications

1. Utilisation des phospholipides cérébraux provenant de cervelles de porc riches en acides gras à longues chaînes polyinsaturées à des doses s'échelonnant de 20 à 40 mg / jour pour la réalisation d'un médicament administré à l'homme atteint d'insuffisance vasculaire cérébrale.

2. Utilisation des phospholipides cérébraux provenant de cervelles de porc riches en acides gras à longues chaînes polyinsaturées à la dose de 40 mg/jour en deux prises pour la réalisation d'un médicament administré à l'homne atteint de présénescence.

3. Utilisation selon la revendication 1 ou 2, dans laquelle les phospholipides cérébraux sont additionnés d'anti-oxydants naturels et/ou d'agents anti-radicalaires.

4. Utilisation selon la revendication 1 ou 2 et la revendication 3, dans laquelle les phospholipides sont additionnés en outre d'huile de poisson.

5. Utilisation selon l'une des revendication 1 à 4, dans laquelle le médicament contient, en outre, des huiles de poisson riches en acide eicosapentoénoïque (EPA) et en acide docosa hexaénoïque (DHA).

6. Utilisation selon l'une des revendications 1 à 5, dans laquelle le médicament contient en outre, un agent antioxydant choisi parmi l'acide ascorbique, les tocophérols et le beta-carotène.

7. Utilisation selon l'une des revendications 1 à 5, dans laquelle le médicament contient en outre, un agent anti-radicalaire choisi parmi le sélénium, la cystéine, la carboxyméthyl cystéine et le diterbutyl paracresol.

8. Utilisation de phospholipides cérébraux provenant de cervelles de porc selon la revendication 1 ou 2 ou la revendication 3, dans laquelle les phospholipides sont associés à des excipients, des diluants ou des véhicules permettant leur administration par voie per os ou par voie intraveineuse.

## Claims

1. Use of brain phospholipids stemming form brains of pig rich in long chain poly unsaturated fatty acids at dosages ranging from 20 to 40 mg/ day for the realization of a drug to be administered to the man suffering from brain vascular.

2. Use of brain phospholipids stemming form porcine brains rich on fatty acids with poly unsaturated log chains, at a doses of 40mg / day with two intakes, in view of the achievement of a drug to be administered to the man suffering from presenescence.

3. Use according to claim 1 or 2, wherein the brain phospholipids are added with natural anti-oxidizing agents and/or anti-radicals agents.

4. Use according to claims 1 or claim 3, wherein the phospholipids are further added with fish oil.

5. Use according to one of the claims 1 to 4, wherein the drug further includes fish oils rich in eicosapentaenoic acid (EPA) and in docosahexaenoic acid (DHA).

6. Use according to one of the claims 1 to 5, wherein the drug further includes an anti-oxidizing agent selected among ascorbic acid, tocopherols and β carotene.

7. Use according to claim one of the claims 1 to 4, wherein the drug further includes an anti-radical agent selected among the selenium, cysteine, carboxymethyl cysteine and diterbutyl paracresol.

8. Use of brain phospholipids stemming from pig brains according to claim 1 or 2 or claim 3 wherein the phospholipids are admixed with carrier, diluents or vehicles allowing their use per oral way or per intravenous way.

## Patentansprüche

1. Verwendung von Hirnsubstanz- Phospholipiden, welche aus dem an mehrfach ungesättigten langkettigen Fettsäuren reichen Gehirnen von Schweinen stammen, in Dosiswerten, die von 20 bis 40 mg / Tag reichen, für die Herstellung eines Arzneimittels, welches Menschen mit cerebraler vascularer Insuffizienz verabreicht wird.

2. Verwendung von Hirnsubstanz Phospholipiden aus dem an mehrfach ungesättigten langkettigen Fettsäuren reichen Gehirnen von Schweinen, in Dosiswerten von 40mg pro Tage bei zwei Abreichungen für die Herstellung eines Arzneimittels, welches Menschen, die an Preseneszenz leiden, verabreicht wird.

3. Verwendung nach Anspruch1, worin den Hirnsubstanz-Phospholipiden natürliche Antioxidationsmittel und/oder antiradikaläre Wirkstoffe zugefügt werden.

4. Verwendung nach Anspruch 1 oder Anspruch 2 und Anspruch 3, worin den Phospholipiden außerdem Fischöl zugefügt wird.

5. Verwendung nach einem der Ansprüche 1 bis 4, worin das Medikament außerdem Fischöl enthält, die reich an Eikosapentaensäure (EPA) und an Dokosahexaensäure (DHA) sind.

6. Verwendung nach einem der Ansprüche 1 bis 5, worin das Arzneimittel außerdem ein Antioxidationsmittel enthält, welches aus Askorbinsäure, Tokopherolen, und Beta-Karotin ausgewählt wird.

7. Verwendung nach einem der Ansprüche 1 bis 5, worin das Arzneimittel einen antiradikalären Wirkstoff enthält, der aus Selen, Zystein, Karboxymethylzistein, und Diterbutylparakresol ausgewählt wird.

8. Verwendung von aus dem Gehirn von Schweinen stammenden Hirnsubstanz Phospholipiden nach Anspruch 1 oder Anspruch 2, bei welcher die Phospolipide an Exzipienten, Verdünnungsmittel oder Trägersubstanzen gebunden sind, welche ihre Verabreichung per os oder auf intravenösen Wege ermöglichen.
